(19) Europäisches Patentamt

European Patent Office

Office européen des brevets

(11) **EP 0 868 906 A1**

(12) ## DEMANDE DE BREVET EUROPEEN

(43) Date de publication:
**07.10.1998 Bulletin 1998/41**

(51) Int Cl.⁶: **A61K 7/42**, A61K 7/48

(21) Numéro de dépôt: **98400634.6**

(22) Date de dépôt: **18.03.1998**

(84) Etats contractants désignés:
**AT BE CH DE DK ES FI FR GB GR IE IT LI LU MC NL PT**
Etats d'extension désignés:
**AL LT LV MK RO SI**

(30) Priorité: **04.04.1997 FR 9704159**

(71) Demandeur: **L'OREAL**
**75008 Paris (FR)**

(72) Inventeurs:
• **Allard, Delphine**
  **92700 Colombes (FR)**
• **Forestier, Serge**
  **77410 Claye Souilly (FR)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(54) **Compositions cosmétiques autobronzantes**

(57)    La présente invention concerne de nouvelles compositions cosmétiques destinées au bronzage artificiel de la peau, comprenant au moins un agent autobronzant, en particulier la DHA, et au moins un polymère choisi parmi les dendrimères.

## Description

La présente invention conceme de nouvelles compositions cosmétiques à usage topique plus particulièrement destinées au bronzage et/ou au brunissage artificiel de la peau (compositions ci-après dénommées plus simplement compositions autobronzantes), ainsi que leur utilisation dans l'application cosmétique susmentionnée. Plus précisément encore, elle concerne des compositions autobronzantes comprenant au moins un agent d'autobronzage, préférentiellement de la dihydroxyacétone, et au moins un polymère choisi parmi les dendrimères, préférentiellement un dendrimère comportant des fonctions amines.

On sait que la dihydroxyacétone, ou DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV. Une telle utilisation présente en outre pour avantage d'éviter totalement les risques de réaction cutanée généralement attachés aux expositions prolongées précitées (érythèmes, brûlures, perte d'élasticité, apparition de rides, vieillissement prématuré de la peau, et autres).

La plupart des produits cosmétiques destinés au bronzage artificiel de la peau utilisent la dihydroxyacétone (DHA) comme actif, mais son utilisation n'est pas entièrement satisfaisante.

En effet, la réaction de la DHA avec les acides aminés de la peau n'est pas immédiate et la coloration cutanée n'apparaît que quelques heures (environ 3 heures) après l'application. En outre, la coloration cutanée conférée par la DHA est peu naturelle car de nuance jaune-orangée.

Diverses solutions ont été proposées afin d'obtenir avec la DHA une coloration plus rapide et d'une nuance plus proche du bronzage naturel.

En particulier, des associations de la DHA avec des composés aminés sont décrites dans plusieurs documents : Les demandes de brevet WO 95/26179 et WO94/22419 décrivent l'association de la DHA avec des acides aminés.

La demande de brevet WO94/04130 décrit l'association de la DHA avec des amines primaires.

Les brevets WO94/13258 et WO 95/15742 décrivent l'association de la DHA avec un composé polyaminé, la polyamine pouvant être par exemple la polyéthylèneimine (polymère hyperbranché) ou une silicone aminée.

Ces associations permettent d'obtenir une montée de la coloration plus rapide par rapport à la DHA seule, mais elles sont loin d'être satisfaisantes en termes de nuance de coloration (jaune-orangée), de tenue de la coloration, en particulier la tenue au lavage, et d'intensité de coloration.

La présente invention a notamment pour but de résoudre les problèmes ci-dessus en proposant de nouvelles associations autobronzantes, en particulier des associations à base de DHA qui présentent une efficacité et/ou une activité autobronzantes sur la peau améliorées : rapidité de coloration, qualité de la coloration, intensité et tenue.

C'est avec étonnement que la demanderesse a constaté que l'association d'un actif autobronzant, en particulier de la dihydroxyacétone ou DHA, avec un dendrimère permettait d'obtenir une coloration artificielle de la peau plus rapidement que la DHA seule et plus rapidement que les autres associations de la DHA avec des composés aminés selon l'art antérieur. Elle permet d'obtenir une nuance plus proche du bronzage naturel. En outre, une telle association confère au bronzage une intensité très satisfaisante et une très bonne tenue dans le temps, en particulier une bonne résistance au lavage.

L'invention a pour objet l'association d'au moins un actif autobronzant et d'au moins un polymère choisi parmi les dendrimères. Plus particulièrement, l'actif autobronzant est la dihydroxyacétone.

Un autre objet de l'invention est constitué par des compositions cosmétiques destinées à une application topique comprenant une telle association.

L'invention concerne également des dispositifs comportant au moins deux compartiments, chacun des compartiments contenant l'un des composants de l'association définie ci-dessus.

D'autres objets de l'invention apparaîtront à la lecture de la description et des exemples qui suivent.

L'actif autobronzant peut être choisi parmi : les dérivés mono ou polycarbonylés, comme la DHA, l'isatine, l'alloxane, la ninhydrine, le glycéraldéhyde, l'aldéhyde mésotartrique, les dérivés de pyrazoline-4,5-dione. L'actif autobronzant préférentiellement utilisé dans la présente invention est la dihydroxyacétone.

Ces actifs autobronzants peuvent être éventuellement associés à des colorants directs ou à des dérivés indoliques.

Les polymères hyperbranchés, catégorie de polymère à laquelle appartient la polyéthylèneimine, sont des constructions moléculaires ayant une structure ramifiée, en général autour d'un coeur. Leur structure est en règle générale dépourvue de symétrie : les unités de base ou monomères ayant servi à la construction du polymère hyperbranché peuvent être de natures différentes et leur répartition est irrégulière. Les branches du polymère peuvent être de natures et de longueurs différentes. Le nombre d'unités de base, ou monomères, peut être différent suivant les différentes ramifications.

Les polymères hyperbranchés sont généralement issus de la polycondensation d'un ou plusieurs monomères $ABx$, A et B étant des groupements réactifs susceptibles de réagir ensemble, x étant un entier supérieur ou égal à 2, mais d'autres procédés de préparation peuvent être envisagés. Les polymères hyperbranchés se caractérisent par leur degré de polymérisation DP = 1-b, b étant le pourcentage de fonctionnalités, non terminales, de B qui n'ont pas réagi avec un groupement A. La condensation étant non systématique, au contraire de la synthèse de dendrimères, le degré de polymérisation est inférieur à 100%. De façon habituelle, par les méthodes de synthèses connues, DP est compris entre 15 et 90%.

Les dendrimères sont des polymères et oligomères hautement ramifiés, également connus en soi, ayant une structure chimique bien définie et on dit que ce sont des polymères hyperbranchés « parfaits ». En règle générale, les dendrimères comprennent un coeur, un nombre déterminé de générations de branches, ou fuseaux, et des groupes terminaux. Les générations de fuseaux sont constituées d'unités structurelles, qui sont identiques pour une même génération de fuseaux et qui peuvent être identiques ou différentes pour des générations de fuseaux différentes. Les générations de fuseaux s'étendent radialement en une progression géométrique à partir du coeur. Les groupes terminaux d'un dendrimère de la $N^{ième}$ génération sont les groupes fonctionnels terminaux des fuseaux de la $N^{ième}$ génération ou génération terminale. De tels polymères sont décrits en particulier dans D.A.Tomalia, A.M.Naylor et W.A. Goddard III, *Angewandte Chemie,* Int.Ed.Engl.29, 138-175 (1990) ; C.J.Hawker et J.M.J.Frechet, *J.Am. Chem. Soc.,* 112, 7638 (1990) ; B.I.Voit, Acta Polymer., 46, 87-99 (1995); N.Ardoin et D.Astruc, Bull. Soc. Chim. Fr. 132, 875-909 (1995) ; G.R.Newkome, C.N.Moorefield, F.Vögtle, Dendritic Molecules, VCH Verlagsgesellschaft, 1996.

On peut également plus particulièrement définir les dendrimères par la formule (DI) suivante :

$$C[A_1B_1(A_2B_2(...(A_{n-1}B_{n-1}(A_nB_n(T)r_n)r_{n-1})r_{n-2}...)r_2)r_1]s \qquad (DI)$$

dans laquelle :

- C représente le coeur, relié par un nombre s de fonctionnalités à s fuseaux $A_1B_1$ par l'intermédiaire des groupements $A_1$ ;

- s est un nombre entier supérieur ou égal à 1 et inférieur ou égal au nombre de fonctionnalités de C ;

- pour chaque fuseau $(A_iB_i)$ (i=1, 2.....n), le groupement $B_i$ est relié à $r_i$ groupements $A_{i+1}$ d'un fuseau $(A_{i+1}B_{i+1})$ ;

- chaque groupement $A_i(i \geq 2)$ est relié à un seul groupement $B_{i-1}$ du fuseau $(A_{i-1}B_{i-1})$;

- $r_1$ (i=1, 2.....n-1) représente le nombre de fonctionnalités du groupement $B_1$ appartenant au fuseau $(A_iB_i)$; ri étant un nombre entier supérieur ou égal à 2

- l'indice i (i=1, 2.....n) est un nombre entier qui désigne la génération de chaque fuseau ;

- le fuseau de $n^{ième}$ génération $A_nB_n$ est chimiquement lié à un nombre $r_n$ de groupes terminaux T ; $r_n$ étant un entier supérieur ou égal à zéro.

La définition des dendrimères donnée ci-dessus inclut des molécules à ramifications symétriques ; elle inclut également des molécules à ramification non symétrique, comme par exemple les dendrimères dont les fuseaux sont des groupements lysine, dans lesquels le branchement d'une génération de fuseaux sur la précédente se fait sur les amines $\alpha$ et $\varepsilon$ de la lysine, ce qui conduit à une différence dans la longueur des fuseaux des différentes ramifications.

Les polymères denses en étoiles, ou «dense star polymer», les polymères éclatés en étoile, ou «starburst polymer», les dendrimères en baguette, ou «rod-shaped dendrimer», sont inclus dans la présente définition des dendrimères. Les molécules dénommées arborols et molécules en cascade entrent également dans la définition des dendrimères selon la présente invention.

Plusieurs dendrimères peuvent être associés entre eux, par une liaison covalente ou un autre type de liaison, par l'intermédiaire de leurs groupes terminaux pour donner des entités connues sous le nom de «dendrimères pontés», «agrégats de dendrimères» ou «bridged dendrimer». De telles entités sont incluses dans la définition des dendrimères selon la présente invention.

Des dendrimères peuvent se présenter sous la forme d'un ensemble de molécules de même génération, ensembles dits monodispersés; ils peuvent également se présenter sous la forme d'ensembles de générations différentes, dits polydispersés. La définition des dendrimères selon la présente invention inclut des ensembles monodispersés aussi bien que polydispersés de dendrimères.

L'invention concerne plus particulièrement les dendrimères à groupements terminaux aminés.

Préférentiellement, en particulier pour des raisons de solubilité, leurs groupements terminaux sont porteurs d'une fonction amine primaire.

On peut se reporter aux documents suivants dans lesquels sont décrits des dendrimères dont le groupe terminal comporte une fonction amine, le contenu de ces documents étant incorporé par référence dans la présente description: US-4,694,064 ; US-4,507,466 ; US-4,631,337 ; US-4,558,120 ; US-4,568,737 ; US-4,587,329 ; WO-A-9502008 ; WO-A-9314147 ; EP-234408 ; US-4,289,872 ; US-4,360,646 ; Proc. Natl. Acad. Sci. USA, 85, 5409-5413 (1988) ; WO95/02008 ; WO93/14147.

Les dendrimères à groupes terminaux aminés sont par exemple des polyamidoamines, comme par exemple ceux commercialisés sous la marque STARBURST PAMAM par la société DENDRITECH (copolymères séquencés d'éthylène diamine et d'acrylate de méthyle). Ils peuvent également être choisis parmi les dendrimères du type polyalkylamine, comme par exemple les polyéthylèneimines et les polypropylèneimines fabriqués par la société DSM.

Les dendrimères à groupes terminaux aminés peuvent également être constitués d'un coeur et de générations d'unités de base, monomères ou fuseaux, de toutes natures, sur lesquels un groupe terminal T porteur d'une fonction amine a été greffé.

De préférence les dendrimères utilisés selon la présente invention comportent des unités de base (monomères ou fuseaux) du type polyamine ou polyamide.

L'association selon l'invention est avantageusement caractérisée par le fait qu'elle comprend :

une première composition (A) comprenant, dans un milieu approprié pour une application topique, au moins un actif auto-bronzant, préférentiellement la dihydroxyacétone, et

une seconde composition (B) comprenant dans un milieu approprié pour une application topique, au moins un polymère choisi parmi les dendrimères.

L'invention a également pour objet un dispositif comprenant un premier compartiment comprenant la composition (A) précitée; un second compartiment comprenant la composition (B) précitée et un moyen de distribution permettant la distribution séquencée ou simultanée des deux compositions.

De tels dispositifs à deux compartiments munis d'un moyen de distribution adapté sont connus en eux-mêmes, par exemple par les documents: WO94/13258 ; WO94/04130 ; WO94/22419 ; FR-2586913.

L'invention a également pour objet une composition comprenant dans un milieu cosmétiquement acceptable au moins un actif auto-bronzant, préférentiellement la dihydroxyacétone et au moins un polymère choisi parmi les dendrimères, tels que définis précédemment.

L'actif auto-bronzant, préférentiellement la dihydroxyacétone ou DHA, est présente dans les compositions selon l'invention dans des proportions suffisantes pour conférer à la peau, après application, une coloration similaire à la coloration obtenue à la suite d'un bronzage naturel. Elle est ainsi généralement présente dans des proportions comprises entre 0,1 et 20%, avantageusement entre 2 et 7% en poids par rapport au poids total de la composition, et de préférence comprises entre 3 et 6% en poids par rapport au poids total de la composition.

Avantageusement le dendrimère est présent dans des proportions allant de 0,01 à 20 %, de préférence de 0,1 à 10 %, en poids par rapport au poids total de la composition.

L'homme du métier saura, par de simples essais, adapter la proportion relative d'actif auto-bronzant et de dendrimère pour obtenir l'effet recherché. Avantageusement, dans les associations selon l'invention, la proportion d'actif auto-bronzant, en particulier de DHA, en poids par rapport au poids de dendrimère, est comprise entre 1/10 et 10, avantageusement entre 1/5 et 5.

Parmi les adjuvants cosmétiques classiques susceptibles d'être contenus dans la phase aqueuse et/ou dans la phase siliconée des émulsions conformes à l'invention (selon leur caractère hydro- et/ou liposoluble), on peut citer notamment les épaississants ioniques ou non ioniques, les adoucissants, les antioxydants, les opacifiants, les stabilisants, les émollients, les agents répulsifs contre les insectes, les filtres solaires organiques actifs dans l'UV-A et/ou l'UV-B, les pigments et les nanopigments minéraux photoprotecteurs, les agents hydratants, les vitamines, les parfums, les conservateurs, les charges, les séquestrants, les colorants, ou tout autre ingrédient habituellement utilisé dans le domaine des produits autobronzants.

Bien entendu, l'homme de l'art veillera à choisir ce ou ces éventuels composés complémentaires et/ou leurs quantités de manière telle que les propriétés avantageuses attachées intrinsèquement à la composition conforme à l'invention ne soient pas, ou substantiellement pas, altérées par la ou les adjonctions envisagées.

Les compositions autobronzantes conformes à l'invention peuvent se présenter sous forme de crèmes, de laits, de gels, de gel-crèmes, de lotions fluides, en particulier de lotions fluides vaporisables, ou tout autre forme généralement utilisée en cosmétique, en particulier celle convenant habituellement aux compositions cosmétiques autobronzantes.

L'invention a en outre pour objet un procédé de traitement non-thérapeutique de la peau, en vue de conférer à celle-ci une coloration proche du bronzage naturel de celle-ci, ce procédé étant caractérisé par le fait que l'on applique sur la peau une quantité efficace d'au moins un actif autobronzant et d'au moins un polymère choisi parmi les dendrimères, soit au sein d'une même composition, soit de façon décalée dans le temps. Lorsque les deux composants sont appliqués au sein d'une même composition, celle-ci est de préférence préparée juste avant l'emploi. Si les deux composants sont appliqués de façon décalée dans le temps on prévoit de préférence d'appliquer le dendrimère en premier lieu puis, de façon immédiate ou jusqu'à quelques heures plus tard, l'actif auto-bronzant.

Un autre objet de la présente invention est constitué par l'utilisation des associations telles que ci-dessus définies comme, ou pour la fabrication de, compositions cosmétiques pour le bronzage et/ou le brunissage artificiels de la peau. Comme indiqué précédemment, les compositions peuvent alors être conditionnées sous la forme de crèmes, de laits, de gels crèmes, ou bien encore de lotions fluides, en particulier de lotions fluides vaporisables, ou tout autre forme appropriée.

L'invention a encore pour objet un produit contenant au moins un actif auto-bronzant, et au moins un polymère choisi parmi les dendrimères comme produit de combinaison pour une utilisation simultanée, séparée ou étalée dans le temps, pour le bronzage artificiel de la peau.

**ESSAIS**

Tous les pourcentages indiqués sont donnés en poids de matière active par rapport au poids total de la composition.

Essais 1 :

Nous avons comparé, au travers d'un test « in vitro » la vitesse d'apparition de la coloration obtenue à partir d'applications successives sur « VITRO-SKIN ™ » (quantité appliquée = 2 mg/cm$^2$) des solutions aqueuses suivantes :

- DHA seule à 10%

- DHA 10% précédée d'une application de solution de lysine à 4 %

- DHA 10% précédée d'une application de solution de polylysine à 4 %

- DHA 10% précédée d'une application de solution de dendrimère (Starburst Pamam de DENDRITECH Génération 5) à 4 %

- DHA 10% précédée d'une application de solution de dendrimère (Starburst Pamam de DENDRITECH Génération 1) à 4 %

Mesures :

- Mesures colorimétriques (système L*a*b) avec Minolta CM-1000R) en fonction du temps avant application (àT$_0$) puis à différents intervalles de temps ;

- Calcul de l'évolution globale de couleur par rapport au T$_0$ :

$$\Delta E = \sqrt{\Delta L^2 + \Delta a^2 + \Delta b^2}$$

Interprétation des mesures: Plus la valeur de $\Delta E$ est élevée , plus l'évolution de la couleur est importante ;

Résultats :

Les valeurs de $\Delta E$ en fonction du temps pour chacun des essais sont représentées sur le graphe en Annexe 1 et montrent clairement une accélération de l'évolution de couleur (donc de l'apparition du bronzage artificiel) lorsque la DHA est associée à un dendrimère (Starburst Pamam de DENDRITECH Génération 5 ou Starburst Pamam de DENDRITECH Génération 1). Cette accélération est beaucoup plus importante qu'avec une association DHA + acide aminé (lysine) ou DHA + polylysine.

Essais 2 :

Nous avons comparé, au travers d'un test « in vivo », la coloration obtenue à partir des protocoles de traitement cosmétique suivants :

P$_1$- Application d'une émulsion H/E préparée à partir des constituants suivants :

- Mélange stéarate de glycérol / alcools gras OE  3 g
- Mélange stéarate de glycérol / stéarate de polyéthylène glycol OE  2.5 g
- Alcool stéarylique  2.5 g
- Huiles de silicone  11 g
- Huiles (esters)  6 g
- Propylène Glycol  5 g
- Dihydroxyacétone  5 g
- Conservateurs  qs
- Eau  qsp  100 g

P$_2$- Application d'une solution (éthanol/propylène glycol/eau : 2/1/1) contenant 5% de polyéthylèneimine (Polymin G-35 commercialisée par la société BASF) puis application de l'émulsion H/E selon P$_1$.

P$_3$- Application d'une solution (éthanol/propylène glycol/eau : 2/1/1) contenant 5% de dendrimère (Starburst Pamam de génération 5 commercialisé par la société DENDRITECH) de puis application de l'émulsion H/E selon P$_1$.

Protocole d'évaluation :

La coloration cutanée obtenue avec l'application des formules décrites ci-dessus a été évaluée au moyen d 'un test « in vivo » sur trois volontaires :

- Région testée :  avant-bras, zones de 2.5 x 2.5 cm
- Quantité appliquée :  2 mg/cm$^2$
- Suivi de l'évolution de la coloration par colorimétrie (Minolta CM-1000R) :

  - avant application (T$_0$)
  - 24H après l'application (T$_{24h}$)

Résultats :

Les résultats sont exprimés dans l'espace couleur L*a*b dans lequel :

- La nuance est exprimée par les paramètres a et b :

  • a représente l'axe rouge-vert (-a=vert, +a=rouge)
  • b représente l'axe jaune-bleu (-b=bleu, +b=jaune)

Nous nous sommes plus particulièrement intéressés au rapport Δa /Δb traduisant l'équilibre rouge / jaune avec :

  • Δa = a(T$_{24H}$) - a (T$_0$)
  • Δb = b (T$_{24H}$) - b(T$_0$)

Sachant que plus la valeur de Δa /Δb est élevée, plus la coloration présente une nuance rouge et que l'on recherche une nuance plus rouge (soit une valeur de Δa /Δb plus élevée) que celle de la DHA seule (trop jaune)

- L'évolution globale de couleur par rapport au T$_0$ avant application est représentée par ΔE :

$$\Delta E = \sqrt{\Delta L^2 + \Delta a^2 + \Delta b^2}$$

Les résultats obtenus sur l'évaluation de la nuance et de l'évaluation globale de couleur sont décrits dans le tableau suivant :

| N° Protocole | $\Delta a / \Delta b$ | $\Delta E$ |
|:---:|:---:|:---:|
| P$_1$ | 0,6 (+/-0,1) | 7,5 (+/-1,4) |
| P$_2$ | 0,4 (+/-0,1) | 4,9 (+/-1,7) |
| P$_3$ | 0,9 (+/-0,4) | 7,5 (+/-0.9) |

Ces résultats montrent que :

- par rapport à la DHA seule, l'association avec le dendrimère permet d'obtenir une nuance moins jaune ($\Delta a / \Delta b$ plus élevé) plus proche d'un bronzage naturel.

- par rapport à l'association DHA + polyéthylèneimine, l'association DHA + dendrimère permet d'obtenir une variation de couleur plus importante (intensité de la coloration plus forte) et une nuance moins jaune, plus équilibrée donc plus naturelle.

**Revendications**

1. Association caractérisée par le fait qu'elle consiste en au moins un actif autobronzant et au moins un polymère choisi parmi les dendrimères.

2. Association selon la revendication 1, caractérisée par le fait que l'actif auto-bronzant est choisi parmi: les dérivés mono ou polycarbonylés, comme la DHA, l'isatine, l'alloxane, la ninhydrine, le glycéraldéhyde, l'aldéhyde mésotartrique, les dérivés de pyrazoline-4,5-dione.

3. Association selon la revendication 2, caractérisée par le fait que l'actif auto-bronzant est la dihydroxyacétone.

4. Association selon l'une quelconque des revendications 1 à 3, caractérisée par le fait qu'elle comporte en outre au moins un composé choisi parmi les colorants directs et les dérivés indoliques.

5. Association selon l'une quelconque des revendications 1 à 4, caractérisée par le fait que le polymère est choisi parmi les dendrimères à groupements terminaux aminés.

6. Association selon l'une quelconque des revendications précédentes, caractérisée par le fait que le polymère est choisi parmi les dendrimères dont les groupements terminaux sont porteurs d'une fonction amine primaire.

7. Association selon la revendication 6, caractérisée par le fait que le dendrimère est choisi parmi les polyamidoamines et polyalkylamines.

8. Association selon l'une quelconque des revendications précédentes, caractérisée par le fait que le dendrimère comporte des unités de base du type polyamine ou polyamide.

9. Association selon l'une quelconque des revendications précédentes, caractérisée par le fait que la proportion d'actif auto-bronzant, en poids par rapport au poids de dendrimère, va de 1/10 à 10, avantageusement de 1/5 à 5.

10. Association selon l'une quelconque des revendications précédentes, caractérisée par le fait qu'elle comprend :

   une première composition (A) comprenant, dans un milieu approprié pour une application topique, au moins un actif auto-bronzant,
   et
   une seconde composition (B) comprenant dans un milieu approprié pour une application topique, au moins un polymère choisi parmi les dendrimères.

11. Composition cosmétique, caractérisée par le fait qu'elle comprend une association selon l'une quelconque des revendications 1 à 10 et un support cosmétiquement acceptable.

12. Composition cosmétique selon la revendication 11, caractérisée par le fait que l'actif auto-bronzant est présent dans des proportions comprises entre 0,1 et 20%, avantageusement entre 2 et 7% en poids par rapport au poids total de la composition, et de préférence comprises entre 3 et 6% en poids par rapport au poids total de la composition.

13. Composition cosmétique selon l'une quelconque des revendications 11 et 12 précédentes, caractérisée par le fait que le dendrimère est présent dans des proportions allant de 0,01 à 20 %, de préférence de 0,1 à 10 %, en poids par rapport au poids total de la composition.

14. Composition cosmétique selon l'une quelconque des revendications 11 à 13 précédentes, caractérisée par le fait qu'elle se présente sous forme d'une crème, d'un lait, d'un gel, d'un gel-crème, d'une lotion fluide, d'une lotion fluide vaporisable.

15. Dispositifs comportant au moins deux compartiments, chacun des compartiments comprenant l'un des composants de l'association selon l'une quelconque des revendication 1 à 10 et un moyen de distribution permettant la distribution séquencée ou simultanée des deux compositions.

16. Utilisation d'une composition selon l'une quelconque des revendications 1 à 14 précédentes comme ou pour la fabrication de compositions destinées au bronzage et/ou au brunissage artificiels de la peau.

17. Procédé de traitement cosmétique de la peau destiné à la bronzer et/ou la brunir artificiellement, caractérisé par le fait qu'il consiste à appliquer sur celle-ci une quantité efficace d'une composition cosmétique telle que définie à l'une quelconque des revendications 1 à 14 précédentes.

18. Produit contenant au moins un actif auto-bronzant, et au moins un polymère choisi parmi les dendrimères comme produit de combinaison pour une utilisation simultanée, séparée ou étalée dans le temps, pour le bronzage artificiel de la peau.

Office européen
des brevets

**RAPPORT DE RECHERCHE EUROPEENNE**

Numéro de la demande

EP 98 40 0634

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,A | WO 95 15742 A (ESTEE LAUDER INC ;LENTINI PETER (US); MARENUS KENNETH (US); MUIZZU) 15 juin 1995 <br> * revendications 1-3,9,13 * <br> --- | 1-3,5,6, 10,15,16 | A61K7/42 A61K7/48 |
| D,A | WO 94 13258 A (SCHERING PLOUGH HEALTHCARE ;MEYER THOMAS A (US); ANDO MICHAEL E (U) 23 juin 1994 <br> * revendications 1,12,22 * <br> --- | 1-3,15, 16 | |
| A | EP 0 684 044 A (DSM NV) 29 novembre 1995 <br> * page 2, ligne 1 - ligne 28 * <br> * page 3, ligne 35 - ligne 38 * <br> * page 4, ligne 38 - ligne 46 * <br> --- | 1 | |
| A | EP 0 671 159 A (SHISEIDO CO LTD) 13 septembre 1995 <br> * exemples * <br> ----- | 1 | |

**DOMAINES TECHNIQUES RECHERCHES (Int.Cl.6)**

A61K

Le présent rapport a été établi pour toutes les revendications

| Lieu de la recherche | Date d'achèvement de la recherche | Examinateur |
|---|---|---|
| BERLIN | 26 juin 1998 | McConnell, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)